# EUROPÄISCHE PATENTSCHRIFT

(11) **EP 1 207 149 B1**
(45) Veröffentlichungstag und Bekanntmachung des Hinweises auf die Patenterteilung: **26.01.2005**
(21) Anmeldenummer: 01125324.2
(22) Anmeldetag: 26.10.2001
(51) Int. Cl.: C07C 209/48, B01J 25/00, C07D 295/12

(54) **Verfahren zur Hydrierung von Nitrilen an Raney-Katalysatoren**
Process for the hydrogenation of nitriles on Raney catalysts
Procédé d'hydrogénation de nitriles sur des catalyseurs de type Raney

(30) Priorität: 16.11.2000 DE 10056839
(43) Veröffentlichungstag der Anmeldung: 22.05.2002
(73) Patentinhaber: BASF AKTIENGESELLSCHAFT, 67056 Ludwigshafen (DE)
(72) Erfinder: Ansmann, Andreas, Dr., 69168 Wiesloch (DE); Benisch, Christoph, Dr., 68165 Mannhcim (DE); Funke, Frank, Dr., 68161 Mannheim (DE); Ohlbach, Frank, Dr., 40219 Düsseldorf (DE); Merger, Martin, Dr., 67227 Frankenthal (DE)
(74) Vertreter: Isenbruck, Günter, Dr.

(56) Entgegenhaltungen:
- EP-A- 0 842 699
- EP-A- 0 880 996
- US-A- 4 895 994

## Beschreibung

Die Erfindung betrifft ein Verfahren zur Hydrierung von Nitrilen zu primären Aminen an einem aktivierten, Makroporen aufweisenden Raney-Katalysator, ein Verfahren zur Herstellung der Raney-Katalysatoren sowie die Raney-Katalysatoren selbst.

Es ist bekannt, Nitrile und Iminonitrile in flüssiger Phase an Raney-Katalysatoren zu hydrieren.

EP-A-0 382 508 beschreibt die halbkontinuierliche Hydrierung von Polynitrilen in flüssiger Phase an Raney-Cobalt-Katalysatoren in Gegenwart von wasserfreiem Ammoniak.

US 5,869,653 beschreibt ein kontinuierliches Verfahren zur Hydrierung von Nitrilen an Raney-Cobalt-Katalysatoren in Abwesenheit von Ammoniak, wobei in Gegenwart katalytischer Mengen von Lithiumhydroxid und Wasser gearbeitet wird.

US 4,895,994 offenbart einen Raney-Katalysator mit einer BET-Oberfläche von 20 bis 80 m²/g und einem Makroporen-Anteil von 0,01 bis 70 Vol.-%, bezogen auf das Gesamtporenvolumen, der hergestellt wird durch Vermischen der Raney-Legierung mit einem hochmolekularen Polymer, Verformen der Mischung zu einem Formkörper, Calcinieren der Zusammensetzung zuerst bei 300 bis 700°C und anschließend bei 850 bis 1200°C in Gegenwart von Sauerstoff und Auslaugen von Aluminium aus dem calcinierten Formkörper durch Behandlung mit 6 N-NaOH bei 90 bis 100°C. Der so aktivierte Katalysator wird anschließend wiederholt mit Wasser gewaschen, bis der pH-Wert des Waschwassers < 9 beträgt. Der Raney-Katalysator wird unter anderem zur Hydrierung von Nitrilen zu Aminen eingesetzt.

EP-A-0 842 699 offenbart ein Verfahren zur Herstellung eines aktivierten, metallpulverfreien, Makroporen aufweisenden Metall-Festbettkatalysators nach Raney auf der Basis einer Legierung aus Aluminium und mindestens einem Metall der VIII. Nebengruppe des Periodensystems, mit den Schritten (1) Herstellen einer Knetmasse enthaltend die Legierung, ein Verformungsmittel, Wasser und einen Porenbildner, (2) Verformen der Knetmasse zu einem Formkörper, (3) Calcinieren des Formkörpers und (4) Behandeln des calcinierten Formkörpers mit einem Alkalimetallhydroxid. Nach der Behandlung des Formkörpers mit Alkalimetallhydroxid wird der aktivierte Katalysator solange mit Wasser gewaschen, bis der pH-Wert des Waschwassers auf 7,5 gesunken ist. Der so erhaltene Katalysator weist einen Gehalt an Makroporen von mehr als 80 Vol.-% auf. Der Katalysator wird zur Hydrierung von Nitrilen zu primären Aminen eingesetzt.

Ein derartiges Verfahren offenbart auch DE-A 44 46 907, wobei Polyvinylalkohol und Wasser oder Stearinsäure als Hilfsmittel eingesetzt werden.

Nachteilig an den Raney-Katalysatoren aus dem Stand der Technik ist, daß sie an ihrer Oberfläche Aluminiumoxide und -hydroxyde mit sauren und/oder basischen Eigenschaften wie Al(OH)₃, AIOOH oder γ-Al₂O₃ aufweisen, die bei der Hydrierung der Nitrile zu Nebenreaktionen führen können. Solche Nebenreaktionen sind beispielsweise die Spaltung des Nitrils oder eine Amin-Imin-Kondensation. Insbesondere für sogenannte Streckernitrile, die durch Anlagerung von Formaldehyd und Blausäure an nukleophile Zentren erhältlich sind, oder sogenannte Michael-Nitrile, die durch Anlagerung von Acrylnitril an nukleophile Zentren erhältlich sind, sind besonders schonende Bedingungen für die Hydrierung notwendig, da diese Nitrile instabil sind und in ihre Edukte zurückspalten können. Zudem weisen einige der beschriebenen Raney-Katalysatoren einen relativ geringen Makroporenanteil auf, so die in US 4,895,994 beschriebenen Katalysatoren, was sich unter dem Gesichtspunkt des Stofftransports im Katalysator negativ auswirkt.

Aufgabe der Erfindung ist es, ein Verfahren zur schonenden Hydrierung von Nitrilen bereitszustellen, das eine hohe Selektivität bezüglich der Bildung primärer Amine aufweist und bei dem Nebenreaktionen wie die Spaltung der Nitrile vermieden werden.

Gelöst wird die Aufgabe durch ein Verfahren zur Hydrierung von Nitrilen zu primären Aminen an einem aktivierten, alpha-Al₂O₃ enthaltenden, Makroporen aufweisenden Raney-Katalysator auf Basis einer Legierung aus Aluminium und mindestens einem Übergangsmetall, ausgewählt aus der Gruppe bestehend aus Eisen, Cobalt und Nickel, und gegebenenfalls einem oder mehreren weiteren Übergangsmetallen, ausgewählt aus der Gruppe bestehend aus Titan, Zirkonium, Chrom und Mangan, der erhältlich ist durch ein Verfahren mit den Schritten in der Reihenfolge (a) - (f):
(a) Herstellen einer Knetmasse enthaltend die Legierung, ein Verformungsmittel, Wasser und einen Porenbildner;
(b) Verformen der Knetmasse zu einem Formkörper;
(c) Calcinieren des Formkörpers;
(d) Aktivieren des calcinierten Formkörpers durch Behandeln mit wässriger Alkalimetallhydroxid-Lösung;
(e) Spülen des Katalysator-Formkörpers mit wässriger Alkalimetallhydroxid-Lösung;
(f) Spülen des Katalysator-Formkörpers mit Wasser.

Die Aufgabe wird ferner gelöst durch ein Verfahren zur Herstellung des Makroporen aufweisenden Raney-Katalysators sowie durch den Makroporen aufweisenden Raney-Katalysator selbst.

Im folgenden wird die Herstellung des erfindungsgemäß eingesetzten Raney-Katalysators genauer beschrieben.

Erfindungsgemäß stellt man zunächst eine Knetmasse aus einer Legierung aus Aluminium und mindestens einem Übergangsmetall, ausgewählt aus der Gruppe bestehend aus Eisen, Cobalt und Nickel, und gegebenenfalls einem oder mehreren weiteren Übergangsmetallen, ausgewählt aus der Gruppe bestehend aus Titan, Zirkonium, Chrom und Mangan, einem Verformungsmittel, Wasser und einem Porenbildner her. Bevorzugte Übergangsmetalle sind Nickel und Cobalt, besonders bevorzugt sind feste Lösungen von Nickel in Cobalt oder Cobalt in Nickel, die das gelöste Metall in einer Konzentration von 0,05 bis 50 Gew.-% enthalten. Zur Erhöhung der Aktivität und Selektivität enthält die Legierung noch mindestens ein weiteres Übergangsmetall, ausgewählt aus Titan, Zirkonium, Chrom und Mangan als Promotor, im allgemeinen in Konzentrationen von 0,01 bis 15 Gew.-%, vorzugsweise 0,05 bis 5 Gew.-%, bezogen auf die Gesamtmenge der Übergangsmetalle. Das Gewichtsverhältnis von Aluminium zu Übergangsmetall liegt in der Regel im Bereich von 30 bis 70 Gew.-% Aluminium und 30 bis 70 Gew.-% Übergangsmetall.

Die Herstellung der Aluminiumlegierung erfolgt in an sich bekannter Weise, beispielsweise wie in DE 21 59 736 beschrieben, deren Inhalt bezüglich der Herstellung von Legierungen aus Aluminium und den genannten Übergangsmetallen durch Bezugnahme in die vorliegende Anmeldung einbezogen wird.

Als Verformungsmittel können alle im Stand der Technik verwendeten Verformungsmittel, wie sie beispielsweise in den US-Patentschriften 4,826,799, 4,895,994, 3,404,551 und 3,358,495 genannt sind, verwendet werden. Vorzugsweise werden Wachse, wie Wachs C Mikropulver PM der HOECHST AG, Fette wie Magnesium- oder Aluminiumstearate, oder Kohlenhydrat enthaltende Polymere, wie Tylose (Methylcellulose), eingesetzt, weiter bevorzugt werden Stearinsäure und Tylose verwendet. Der Gehalt des Verformungsmittels in der Knetmasse beträgt im allgemeinen ungefähr 0,1 bis ungefähr 3 Gew.-%, vorzugsweise ungefähr 0,2 bis ungefähr 2 Gew.-%, bevorzugt ungefähr 0,5 bis ungefähr 1 Gew.-%.

Als Porenbildner können Polymere eingesetzt werden, sofern sie eine Molmasse von mehr als 6000 bis ungefähr 500 000 g/mol aufweisen. Ihr Molekulargewicht beträgt vorzugsweise ungefähr 10 000 bis ungefähr 200 000 g/mol, weiter bevorzugt ungefähr 13 000 bis ungefähr 150 000 g/mol und insbesondere ungefähr 13 000 bis ungefähr 50 000 g/mol.

Beispiele für im Rahmen des erfindungsgemäßen Verfahrens als Porenbildner verwendbare Polymere sind Polyvinylchlorid, Copolymere eines Olefins mit polaren Comonomeren, wie Ethylen oder Propylen mit Polyvinylchlorid, Polyvinylidenchloridcopolymere, ABS-Harze, Polyethylencopolymere mit Vinylacetat, Alkylacrylate, Acrylsäure, chlorierte Polyethylene, chlorosulfonierte Polyethylene, thermoplastische Polyurethane, Polyamide, wie Nylon-5, Nylon-12, Nylon-6/6, Nylon-6/10, Nylon-11, Fluor enthaltende Harze, wie FEP, Polyvinylidenfluorid, Polychlortrifluorethylen, Acrylnitril-Methyl(meth)acrylat-Copolymere, Acrylnitril-Vinylchlorid-Copolymere, Styrol-Acrylnitril-Copolymere, wie Methacrylnitril-Styrol-Copolymere, Polyalkyl(meth)acrylate, Celluloseacetat, Celluloseacetatbutyrat, Polycarbonate, Polysulfone, Polyphenylenoxid, Polyester, wie Butylenterephthalat, und Polyvinylalkohol, wobei Polyvinylalkohol besonders bevorzugt ist. In Frage kommen ferner Polyethylenoxide und Kartoffelstärke.

Das Gehalt des Porenbildners in der Knetmasse beträgt ungefähr 1 bis ungefähr 20 Gew.-%, bevorzugt ungefähr 4 bis ungefähr 8 Gew.-%, jeweils bezogen auf das Gesamtgewicht der Knetmasse.

Die Knetmasse aus der Legierung, dem Verformungsmittel, Wasser und dem Porenbildner wird zu Formkörpern geformt, wobei Tabletten und Stränge bevorzugt sind. Die Verarbeitung zu den Formkörpern wird in hierfür bekannten Apparaturen, beispielsweise in Extrudern, Tabletten- oder Strangpressen, durchgeführt.

Im allgemeinen wird zunächst die Legierung mit dem Verformungsmittel und dem üblicherweise festen Polymer als Porenbildner vermischt und anschließend portionsweise so lange Wasser zugegeben, bis eine gut formbare, plastische Masse erhalten wird. Zur Herstellung derartiger Knetmassen verwendet man die üblicherweise verwendeten Mischoder Knetvorrichtungen.

In einer besonderen Ausführungsform werden Stränge mit einem Durchmesser von 3,0 mm hergestellt, die in der Regel nach dem Austreten aus dem Extruder bei Raumtemperatur antrocknen gelassen werden. Anschließend erfolgt eine Trocknung bei 80 bis 200°C über 12 bis 24 Stunden.

Die Calcinierung der Formkörper wird vorzugsweise als besonders schonender dreistufiger Calcinierungsprozeß bei Atmosphärendruck durchgeführt. Dabei werden die Formkörper vorzugsweise zunächst 1 bis 3 Stunden bei ungefähr 400 bis ungefähr 500°C, danach 1 bis 3 Stunden bei ungefähr 700 bis ungefähr 800°C und anschließend bei 1 bis 3 Stunden bei ungefähr 900 bis ungefähr 1000°C behandelt. Die Calcinierung wird üblicherweise an Luft vorgenommen.

Die Aktivierung der calcinierten Formkörper wird erfindungsgemäß mit einem Alkalimetallhydroxid, vorzugsweise Lithiumhydroxid, Natriumhydroxid, Kaliumhydroxid oder Cäsiumhydroxid oder deren Gemischen, besonders bevorzugt mit Natriumhydroxid allein oder im Gemisch mit den oben genannten Alkalimetallhydroxiden, durchgeführt. In der Regel werden wässrige Lösungen des Alkalimetallhydroxids, bevorzugt Natronlauge, verwendet, wobei die Alkalilauge 5 bis 30 gew.-%ig, vorzugsweise 15 bis 25 gew.-%ig ist. Das Molverhältnis von Alkalimetallhydroxid zu Aluminium beträgt in der Regel ungefähr 1 : 1 bis ungefähr 5 : 1, vorzugsweise ungefähr 1,5 : 1 bis ungefähr 3 : 1.

Die Temperatur der Aktivierung beträgt üblicherweise ungefähr 25°C bis ungefähr 106°C, vorzugsweise ungefähr 45°C bis ungefähr 90°C.

Die Dauer der Aktivierung hängt im wesentlichen vom gewünschten Endgehalt an Aluminium ab und liegt im allgemeinen im Bereich von 1 bis 10 Stunden, vorzugsweise 2 bis 5 Stunden. Die Aktivierungsprozedur kann auch mehrfach durchgeführt werden.

Nach der Aktivierung wird der Katalysator-Formkörper mit wässriger Alkalimetallhydroxid-Lösung und anschließend mit Wasser gespült. Wesentlich ist, daß vor dem Spülen mit Wasser zunächst mit wässriger Alkalimetallhydroxid-Lösung gespült wird.

Im allgemeinen wird mit einer wässrigen Lösung des Alkalimetallhydroxids gespült, wobei die Alkalilauge 5 bis 30 gew.-%ig, vorzugsweise 15 bis 25 gew.-%ig ist. In einer bevorzugten Ausführungsform der Erfindung wird zum Spülen das gleiche Alkalimetallhydroxid wie bei der Aktivierung des Katalysators eingesetzt, und ist die Alkalimetallhydroxid-Konzentration der Spüllösung die gleiche wie in der zur Aktivierung eingesetzten Alkalimetallhydroxid-Lösung. Das Spülen wird vorzugsweise bei Raumtemperatur durchgeführt. Dabei kann das Spülen mit Alkalimetallhydroxid-Lösung mehrfach durchgeführt werden, vorzugsweise wird es 3-fach durchgeführt, wobei die Gesamtmenge an eingesetzter Lauge ähnlich groß ist wie bei der Aktivierung. Erst danach erfolgt das Spülen mit Wasser, vorzugsweise solange, bis ein pH-Wert des Spülwassers von ca. 8 erreicht ist.

Die so hergestellten Katalysatoren weisen einen alpha-Al₂O₃-Gehalt von im allgemeinen > 0,1 Gew.-%, bevorzugt > 1,0 Gew.-% auf.

Die gewaschenen, aktivierten Katalysator-Formkörper werden unter Wasser, vorzugsweise in einer Mischung aus Wasser und Methanol aufbewahrt.

Die so hergestellten Katalysator-Formkörper weisen ein Makroporenvolumen von mindestens 0,1 ml/g, bevorzugt von 0,1 - 0,5 ml/g, einen Makroporenanteil von mindestens 80 Vol.-%, bevorzugt von 85 bis 95 Vol.-%, bezogen auf das Gesamtporenvolumen, und eine spezifische Oberfläche von ≤ 20 m²/g, bevorzugt von 10 bis 20 m²/g, auf. Unter Makroporen werden erfindungsgemäß Poren mit einem Durchmesser ≥ 50 nm verstanden.

Erfindungsgemäß können beliebige Nitrile zu den entsprechenden primären Aminen hydriert werden. Bevorzugte Nitrile, die nach dem erfindungsgmäßen Verfahren hydriert werden, sind sogenannte Streckernitrile und Nitrile, die durch Michael-Addition erhalten werden können, der allgemeinen Formeln (I) bzw. (II) 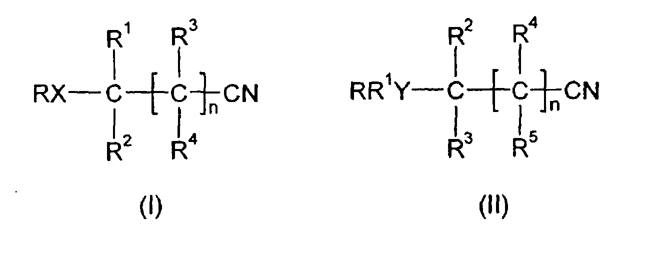 worin
- X: O oder S,
- Y: N oder P,
- n: 0 oder 1
und
- R, R¹, R², R³, R⁴, R⁵: unabhängig voneinander H oder einen gegebenenfalls substituierten Alkylrest mit 1 - 10 C-Atomen oder einen gegebenenfalls substituierten Arylrest mit 6 - 12 C-Atomen
bedeuten.

Beispiele sind Iminodiacetonitril, Nitrilotrisacetonitril, Ethylendiamintetraacetonitril, Biscyanoethylpiperazin, Dimethylaminopropionitril Dimethylaminopropylaminopropionitril und Methoxypropionitril.

Bevorzugte Nitrile sind ferner cyclische Iminonitrile und acyclische Iminonitrile der allgemeinen Formeln (III) und (IV) 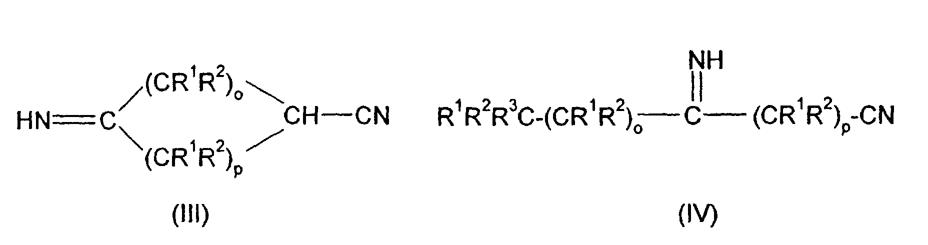 worin
- R¹, R² und R³: unabhängig voneinander H oder einen gegebenenfalls substituierter Alkylrest mit 1 - 10 C-Atomen oder ein gegebenenfalls substituierter Arylrest mit 6 - 12 C-Atomen und
- o,p: 0, 1, 2, 3, 4 oder 5
bedeuten.

Ein Beispiel ist Isophoronnitrilimin.

Weitere wichtige Nitrile und Aminonitrile, die nach dem erfindungsgemäßen Verfahren hydriert werden können, sind die nachstehenden Verbindungen (V) bis (XXXII):

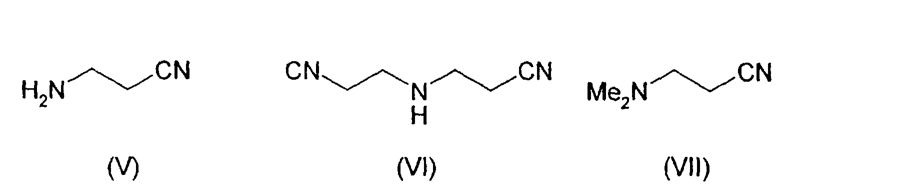

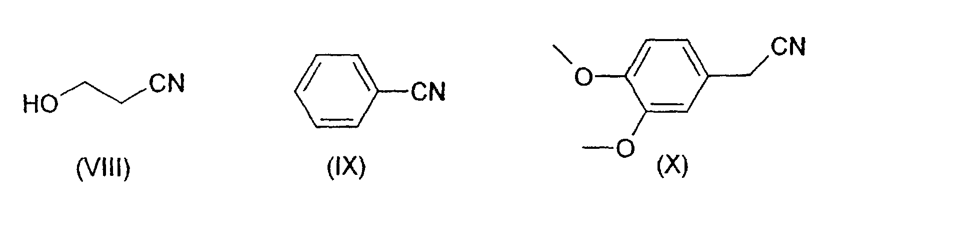

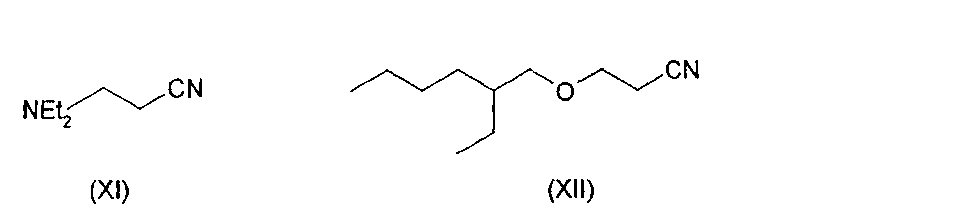

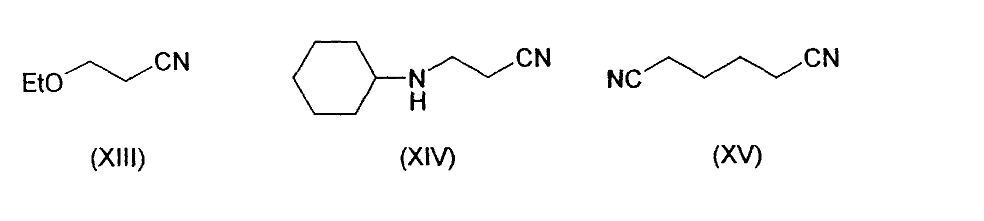

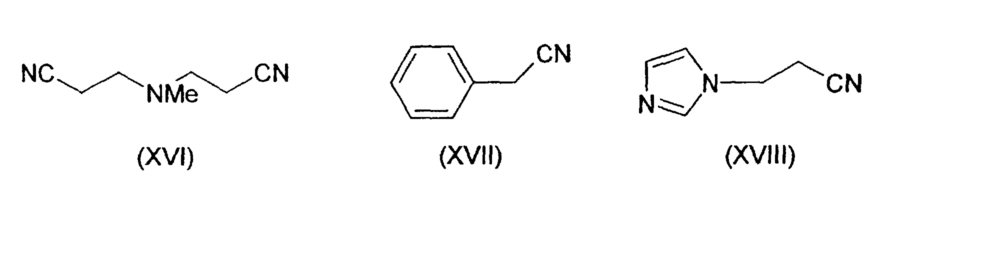

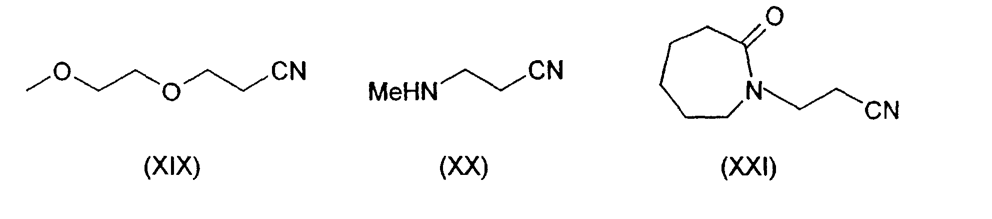

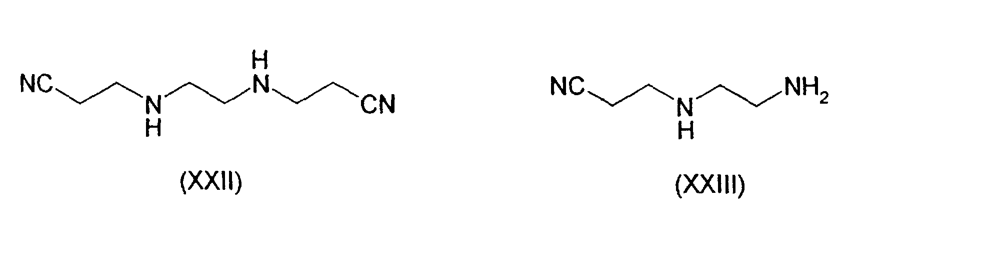

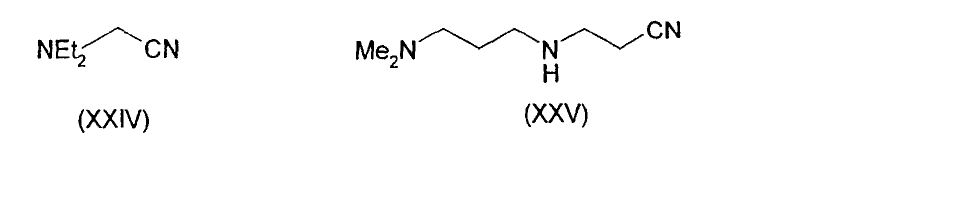

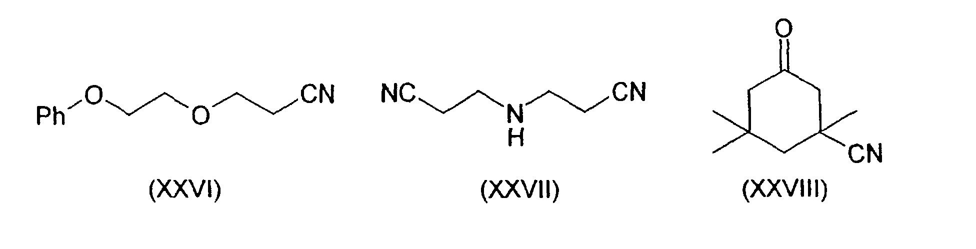

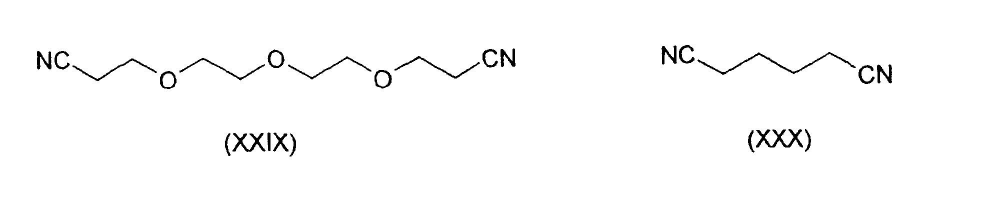

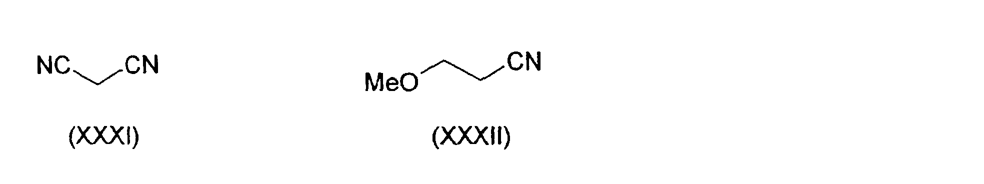

Die Hydrierung kann in der Gasphase, mit oder ohne Lösungsmittel, kontinuierlich als Festbettreaktion mit fest angeordnetem Katalysator, beispielsweise in Sumpf- oder Rieselfahrweise, oder als Wirbelbettreaktion mit in auf- und abwirbelnder Bewegung befindlichem Katalysator durchgeführt werden. Bevorzugt ist das Arbeiten im Festbett. Dinitrile oder Polynitrile können auch teilweise hydriert werden. Durch Veränderung der Verweilzeit können der Umsatz und damit die Produktverhältnisse von Aminen zu Aminonitrilen gezielt eingestellt werden.

Die Hydrierung kann an einem Katalysatorfestbett durchgeführt werden. Geeignete Reaktoren für die Hydrierung in Festbett-Fahrweise sind Rohrreaktoren. Die Temperatur beträgt im allgemeinen von 25 bis 125°C, der Wasserstoffdruck im allgemeinen von 10 bis 300 bar. Die zu hydrierenden Nitrile können in einem organischen Lösungsmittel gelöst vorliegen. Bevorzugte organische Lösungsmittel sind aliphatische Alkohole, Amine, Amide wie N-Methylpyrrolidon und Dimethylformamid, Ether oder Ester. Die Katalysatorbelastung liegt im allgemeinen zwischen 0,5 und 20, vorzugsweise zwischen 2 und 10 Mol Nitril/l_{Katalysator}*h. Die Hydrierung in Festbett-Fahrweise kann in Gegenwart von Ammoniak durchgeführt werden. Der Ammoniakgehalt beträgt im allgemeinen von 6 bis 60, bevorzugt von 12 bis 36 Mol Ammoniak pro Mol des zu hydrierenden Nitrils.

Die Erfindung wird durch die nachstehenden Beispiele näher erläutert.

### Beispiele

### Beispiel 1

### Herstellung eines dotierten Aluminium-Cobalt-Festbettkatalysators

Zu einer Mischung aus 900 g eines Pulvers, bestehend aus einer Legierung aus 49,1 Gew.-% Aluminium, 47 Gew.-% Cobalt, 1,5 Gew.-% Chrom, 1,7 Gew.-% Nickel und 0,28 Gew.-% Eisen, 80 g Polyvinylalkohol mit einem Molekulargewicht zwischen 9000 und 18000 g/mol und 8 g Tylose (H2000 P der Fa. Clariant) werden portionsweise 130 ml Wasser in einem Kneter hinzugefügt. Nach einer Knetzeit von drei Stunden wird die plastische Masse bei einem Preßdruck von 14 MPa in einer Strangpresse zu 3 mm Strängen verformt. Die Stränge werden 16 h an Luft getrocknet, danach 16 h bei 120°C getrocknet und anschließend 1 h bei 450°C, 1 h bei 750°C und 2 h bei 900°C calciniert. Danach werden die Stränge zu Splitt mit einer Kornfraktion von 1,5 - 4 mm zerkleinert. 300 g Splitt werden zu 4 l 20 gew.-%iger Natronlauge bei 70°C gegeben. Nach Abklingen der Wasserstoffentwicklung nach ca. 1 h wird auf 90°C aufgeheizt und für 3 h bei dieser Temperatur gehalten. Der so aktivierte Katalysator wird durch Abdekantieren von der zur Aktivierung verwendeten Natronlauge getrennt. Danach werden 1000 ml einer frischen 20 gew.-%igen Natronlauge dem Katalysator bei Raumtemperatur hinzugefügt. Nach fünfminütigem Rühren wird wiederum abdekantiert. Dieses Waschen mit Natronlauge wird noch zweimal wiederholt. Anschließend wird der Katalysator mit Wasser gewaschen, bis das Waschwasser einen pH-Wert von ca. 8 aufweist.

Es wird ein Katalysator mit einer spezifischen Oberfläche nach BET von 18,3 m²/g, einem Porenvolumen von 0,26 ml/g, einem Makroporenanteil von 94 Vol.-% und einem α-Al₂O₃-Gehalt von > 1 Gew.-% erhalten.

### Beispiel 2

### Hydrierung von Imidodiacetonitril zu Diethylentriamin

120 g des nach Beispiel 1 hergestellten, wasserfeuchten Katalysators werden in einen kontinuierlich betriebenen 100 ml-Laborreaktor eingebaut und der Reaktor wird mit Stickstoff gespült. Anschließend wird der Reaktor mit 40 bar Ammoniak geflutet. Es werden 20 Nl/h Wasserstoff und 18 g/h Ammoniak auf 100 bar aufgepreßt. Anschließend werden die Temperatur auf 60°C und der Druck auf 200 bar erhöht. Es werden 0,05 kg/l (Katalysator)*h Imidodiacetonitril, das als 20 gew.-%ige Lösung in N-Methylpyrrolidon eingesetzt wird, hydriert. Bei einem Umsatz von 99 % werden 89 Gew.-% Diethylentriamin gebildet. Hauptnebenprodukt sind 7 Gew.-% Piperazin.

### Beispiel 3

### Hydrierung von Biscyanoethylpiperazin zu Bisaminopropylpiperazin

120 g des nach Beispiel 1 hergestellten, wasserfeuchten Katalysators werden in einen kontinuierlich betriebenen 100 ml-Laborreaktor eingebaut und der Reaktor mit Stickstoff gespült. Anschließend wird der Reaktor mit 40 bar Ammoniak geflutet und es werden 100 Nl/h Wasserstoff und 51 g/h Ammoniak auf 65 bar aufgepreßt. Die Temperatur wird auf 80°C erhöht. Es werden 0,15 kg/l(Katalysator)*h Biscyanoethylpiperazin, das als 50 gew.-%ige Lösung in Wasser eingesetzt wird, hydriert. Bei einem Umsatz von 99,5 % werden 97,2 Gew.-% Bisaminopropylpiperazin gebildet. Hauptnebenprodukte sind das Mononitril (0,5 Gew.-%) und Aminopropylpiperazin (1,2 Gew.-%).

Bei einer Belastung von 0,2 kg/l(Katalysator)*h und einer Hydrierungstemperatur von 100°C wird bei einem Umsatz von 97,7 % 95,8 Gew.-% Bisaminopropylpiperazin erhalten. Hauptnebenprodukte sind das Mononitril (1,5 Gew.-%) und Aminopropylpiperazin (0,7 Gew.-%).

### Beispiel 4

### Hydrierung von Isophoronnitril zu Isophorondiamin

In einer kontinuierlich betriebenen Laborapparatur, bestehend aus einem ersten Reaktor, der mit 100 ml Al₂O₃ (4 mm-Stränge) gefüllt ist, und einem zweiten Reaktor, der mit 240 g (200 ml) des nach Beispiel 1 hergestellten Raney-Katalysators gefüllt ist, werden 32 ml/h Isophoronnitril mit 300 ml/h H₂ in Gegenwart von 147 ml/h NH₃ bei einem Druck von 250 bar und einer Temperatur von 250°C hydriert. Die Katalysator-Belastung beträgt 0,15 kg/l(Katalysator)*h, die Verweilzeit beträgt 44 min. Bei einem Umsatz von 99,2% wird Isophorondiamin mit einer Selektivität von 89,5% erhalten, wobei das cis/trans-Isomerenverhältnis 79 : 21 beträgt.

## Patentansprüche

1. Verfahren zur Hydrierung von Nitrilen zu primären Aminen an einem aktivierten, alpha-Al₂O₃ enthaltenden, Makroporen aufweisenden Raney-Katalysator auf Basis einer Legierung aus Aluminium und mindestens einem Übergangsmetall, ausgewählt aus der Gruppe bestehend aus Eisen, Cobalt und Nickel, und gegebenenfalls einem oder mehreren weiteren Übergangsmetallen, ausgewählt aus der Gruppe bestehend aus Titan, Zirkonium, Chrom und Mangan, der erhältlich ist durch ein Verfahren mit den Schritten in der Reihenfolge (a) - (f):
(a) Herstellen einer Knetmasse enthaltend die Legierung, ein Verformungsmittel, Wasser und einen Porenbildner;
(b) Verformen der Knetmasse zu einem Formkörper;
(c) Calcinieren des Formkörpers;
(d) Aktivieren des calcinierten Formkörpers durch Behandeln mit wässriger Alkalimetallhydroxid-Lösung;
(e) Spülen des Katalysator-Formkörpers mit wässriger Alkalimetallhydroxid-Lösung;
(f) Spülen des Katalysator-Formkörpers mit Wasser.

2. Verfahren nach Anspruch 1, **dadurch gekennzeichnet, dass** der Katalysator auf Basis einer Legierung aus Aluminium und einer festen Lösung aus Nickel in Cobalt oder Cobalt in Nickel, die 0,05 - 50 Gew.-% des gelösten Metalls und gegebenenfalls 0,05 bis 5 Gew.-% des oder der weiteren Übergangsmetalle enthält, hergestellt ist.

3. Verfahren nach Anspruch 1 oder 2, **dadurch gekennzeichnet, daß** der Makroporenanteil des Katalysators mindestens 80 % und das Gesamtporenvolumen mindestens 0,1 ml/g beträgt.

4. Verfahren nach einem der Ansprüche 1 bis 3, **dadurch gekennzeichnet, dass** die Hydrierung kontinuierlich an einem Katalysator-Festbett durchgeführt wird.

5. Verfahren nach Anspruch 4, **dadurch gekennzeichnet, dass** die Hydrierung in einem Strömungsrohr in Sumpf- oder Rieselfahrweise durchgeführt wird.

6. Verfahren nach einem der Ansprüche 1 bis 5, **dadurch gekennzeichnet, daß** das zu hydrierende Nitril in einem organischen Lösungsmittel gelöst vorliegt.

7. Verfahren nach Anspruch 6, **dadurch gekennzeichnet, daß** als Lösungsmittel Dimethylformamid oder N-Methylpyrrolidon eingesetzt wird.

8. Verfahren nach einem der Ansprüche 1 bis 7, **dadurch gekennzeichnet, daß** die Hydrierung in Gegenwart von Ammoniak durchgeführt wird.

9. Verfahren nach einem der Ansprüche 1 bis 8, **dadurch gekennzeichnet, daß** die Hydrierung bei einer Temperatur von 25 bis 125°C und einem Druck von 10 bis 300 bar durchgeführt wird.

10. Verfahren nach einem der Ansprüche 1 bis 9, **dadurch gekennzeichnet, daß** das Nitril ausgewählt ist aus Strecker-Nitrilen oder Michael-Addukten an Acrylniril.

11. Verfahren nach einem der Ansprüche 1 bis 9, **dadurch gekennzeichnet, daß** das Nitril ausgewählt ist aus der Gruppe bestehend aus Iminodiacetonitril, Nitrilotrisacetonitril, Ethylendiamintetraacetonitril, Biscyanoethylpiperazin, Dimethylaminopropionitril, Dimethylaminopropylaminopropionitril, Isophoronnitrilimin und Methoxypropionitril.

12. Verfahren zur Herstellung eines aktivierten, alpha-Al₂O₃ enthaltenden, Makroporen aufweisenden Raney-Katalysator auf Basis einer Legierung aus Aluminium und mindestens einem Übergangsmetall, ausgewählt aus der Gruppe bestehend aus Eisen, Cobalt und Nickel, und gegebenenfalls einem oder mehreren weiteren Übergangsmetallen, ausgewählt aus der Gruppe bestehend aus Titan, Zirkonium, Chrom und Mangan, mit den Schritten in der Reihenfolge (a) - (f):
(a) Herstellen einer Knetmasse enthaltend die Legierung, ein Verformungsmittel, Wasser und einen Porenbildner;
(b) Verformen der Knetmasse zu einem Formkörper;
(c) Calcinieren des Formkörpers;
(d) Aktivieren des calcinierten Formkörpers durch Behandeln mit wässriger Alkalimetallhydroxid-Lösung;
(e) Spülen des Katalysator-Formkörpers mit wässriger Alkalimetallhydroxid-Lösung;
(f) Spülen des Katalysator-Formkörpers mit Wasser.

13. Makroporen aufweisender Raney-Katalysator, herstellbar nach dem Verfahren gemäß Anspruch 12.

14. Makroporen aufweisender Raney-Katalysator nach Anspruch 13, **dadurch gekennzeichnet, daß** der Makroporenanteil mindestens 80 % und das Gesamtporenvolumen mindestens 0,1 ml/g beträgt.

## Claims

1. A process for hydrogenating nitriles to primary amines over an activated, alpha-Al₂O₃-containing, macroporous Raney catalyst based on an alloy of aluminum and at least one transition metal selected from the group consisting of iron, cobalt and nickel, and, if desired, one or more further transition metals selected from the group consisting of titanium, zirconium, chromium and manganese, which is obtainable by a process comprising the steps in the order (a)-(f):
(a) preparing a kneadable composition comprising the alloy, a shaping aid, water and a pore former;
(b) shaping the kneadable composition to form a shaped body;
(c) calcining the shaped body;
(d) activating the calcined shaped body by treatment with aqueous alkali metal hydroxide solution;
(e) rinsing the shaped catalyst body with aqueous alkali metal hydroxide solution;
(f) rinsing the shaped catalyst body with water.

2. A process as claimed in claim 1, wherein the catalyst has been produced on the basis of an alloy of aluminum and a solid solution of nickel in cobalt or of cobalt in nickel which comprises 0.05-50% by weight of the dissolved metal and, if desired, from 0.05 to 5% by weight of one or more of the further transition metals.

3. A process as claimed in claim 1 or 2, wherein the proportion of macropores in the catalyst is at least 80% and the total pore volume is at least 0.1 ml/g.

4. A process as claimed in any of claims 1 to 3, wherein the hydrogenation is carried out continuously over a fixed bed of catalyst.

5. A process as claimed in claim 4, wherein the hydrogenation is carried out in the upflow or downflow mode in a flow tube.

6. A process as claimed in any of claims 1 to 5, wherein the nitrile to be hydrogenated is present as a solution in an organic solvent.

7. A process as claimed in claim 6, wherein the solvent used is dimethylformamide or N-methylpyrrolidone.

8. A process as claimed in any of claims 1 to 7, wherein the hydrogenation is carried out in the presence of ammonia.

9. A process as claimed in any of claims 1 to 8, wherein the hydrogenation is carried out at from 25 to 125°C and a pressure of from 10 to 300 bar.

10. A process as claimed in any of claims 1 to 9, wherein the nitrile is selected from the group consisting of extender nitriles and Michael adducts of acrylonitrile.

11. A process as claimed in any of claims 1 to 9, wherein the nitrile is selected from the group consisting of iminodiacetonitrile, nitrilotriacetonitrile, ethylenediaminetetraacetonitrile, biscyanoethylpiperazine, dimethylaminopropionitrile, dimethylaminopropylaminopropionitrile, isophoronenitrilimine and methoxypropionitrile.

12. A process for producing an activated, alpha-Al₂O₃- containing, macroporous Raney catalyst based on an alloy of aluminum and at least one transition metal selected from the group consisting of iron, cobalt and nickel, and, if desired, one or more further transition metals selected from the group consisting of titanium, zirconium, chromium and manganese, which comprises the steps in the order (a)-(f):
(a) preparing a kneadable composition comprising the alloy, a shaping aid, water and a pore former;
(b) shaping the kneadable composition to form a shaped body;
(c) calcining the shaped body;
(d) activating the calcined shaped body by treatment with aqueous alkali metal hydroxide solution;
(e) rinsing the shaped catalyst body with aqueous alkali metal hydroxide solution;
(f) rinsing the shaped catalyst body with water.

13. A macroporous Raney catalyst which can be produced by a process as claimed in claim 12.

14. A macroporous Raney catalyst as claimed in claim 13 having a proportion of macropores of at least 80% and a total pore volume of at least 0.1 ml/g.

## Revendications

1. Procédé d'hydrogénation de nitriles en amines primaires sur un catalyseur de Raney activé, qui contient de l'alpha-Al₂O₃ et présente des macropores et qui est à base d'un alliage d'aluminium et d'au moins un métal de transition, choisi parmi le groupe constitué du fer, du cobalt et du nickel, et éventuellement d'un ou plusieurs autres métaux de transition, choisis parmi le groupe constitué du titane, du zirconium, du chrome et du manganèse, catalyseur qui peut être obtenu par un procédé présentant, dans l'ordre (a)-(f), les étapes :
(a) de préparation d'une masse de malaxage contenant l'alliage, un agent de déformation, de l'eau et un agent porogène,
(b) de déformation de la masse de malaxage en un corps façonné,
(c) de calcination du corps façonné,
(d) d'activation du corps façonné calciné par traitement par une solution aqueuse d'hydroxyde de métal alcalin,
(e) de rinçage du corps façonné de catalyseur par une solution aqueuse d'hydroxyde de métal alcalin,
(f) de rinçage du corps façonné de catalyseur par de l'eau.

2. Procédé suivant la revendication 1, **caractérisé en ce que** le catalyseur à base d'un alliage est préparé à partir d'aluminium et d'une solution solide de nickel dans du cobalt ou de cobalt dans du nickel, qui contient 0,05-50 % en poids du métal dissous et éventuellement 0,05 à 5 % en poids du ou des autres métaux de transition.

3. Procédé suivant l'une des revendications 1 et 2, **caractérisé en ce que** la fraction de macropores du catalyseur est d'au moins 80 % et **en ce que** le volume poreux total est d'au moins 0,1 ml/g.

4. Procédé suivant l'une des revendications 1 à 3, **caractérisé en ce que** l'hydrogénation est effectuée de manière continue sur un lit fixe de catalyseur.

5. Procédé suivant la revendication 4, **caractérisé en ce que** l'hydrogénation est effectuée dans un tube d'écoulement suivant un mode de fonctionnement en fond de cuve ou à ruissellement.

6. Procédé suivant l'une des revendications 1 à 5, **caractérisé en ce que** le nitrile à hydrogéner se présente en solution dans un solvant organique.

7. Procédé suivant la revendication 6, **caractérisé en ce que**, comme solvant, on met en oeuvre du diméthylformamide ou de la N-méthylpyrrolidone.

8. Procédé suivant l'une des revendications 1 à 7, **caractérisé en ce que** l'hydrogénation est effectuée en présence d'ammoniac.

9. Procédé suivant l'une des revendications 1 à 8, **caractérisé en ce que** l'hydrogénation est effectuée à une température de 25 à 125°C et à une pression de 10 à 300 bars.

10. Procédé suivant l'une des revendications 1 à 9, **caractérisé en ce que** le nitrile est choisi parmi des nitriles de Strecker ou des produits d'addition de Michael sur de l'acrylonitrile.

11. Procédé suivant l'une des revendications 1 à 9, **caractérisé en ce que** le nitrile est choisi parmi le groupe constitué d'iminodiacétonitrile, de nitrilotriacétonitrile, d'éthylènediaminotétraacétonitrile, de biscyanoéthylpipérazine, de diméthylaminopropionitrile, de diméthylaminopropylaminopropionitrile, d'isophoronenitrilimine et de méthoxypropionitrile.

12. Procédé de préparation d'un catalyseur de Raney activé qui contient de l'alpha-Al₂O₃ et présente des macropores et qui est à base d'un alliage d'aluminium et d'au moins un métal de transition, choisi parmi le groupe constitué du fer, du cobalt et du nickel, et éventuellement d'un ou plusieurs autres métaux de transition, choisis parmi le groupe constitué du titane, du zirconium, du chrome et du manganèse, ce procédé comprenant, dans l'ordre (a)-(f), les étapes :
(a) de préparation d'une masse de malaxage contenant l'alliage, un agent de déformation, de l'eau et un agent porogène,
(b) de déformation de la masse de malaxage en un corps façonné,
(c) de calcination du corps façonné,
(d) d'activation du corps façonné calciné par traitement par une solution aqueuse d'hydroxyde de métal alcalin,
(e) de rinçage du corps façonné de catalyseur par une solution aqueuse d'hydroxyde de métal alcalin,
(f) de rinçage du corps façonné de catalyseur par de l'eau.

13. Catalyseur de Raney présentant des macropores, que l'on peut préparer suivant le procédé selon la revendication 12.

14. Catalyseur de Raney présentant des macropores suivant la revendication 13, **caractérisé en ce que** la fraction de macropores est d'au moins 80 % et **en ce que** le volume poreux total est d'au moins 0,1 ml/g.
